Europäisches Patentamt

(19) European Patent Office

Office européen des brevets

(11) Publication number : **0 297 213 B1**

(12)

# EUROPEAN PATENT SPECIFICATION

(45) Date of publication of patent specification :
**20.02.91 Bulletin 91/08**

(51) Int. Cl.⁵ : **A61K 7/16, A61K 7/18**

(21) Application number : **88103996.0**

(22) Date of filing : **14.06.83**

(54) Oral treatment and use of an oral composition.

Divisional application 89100014.3 filed on
14/06/83.

(30) Priority : **22.06.82 US 391040**

(43) Date of publication of application :
**04.01.89 Bulletin 89/01**

(45) Publication of the grant of the patent :
**20.02.91 Bulletin 91/08**

(84) Designated Contracting States :
**AT BE CH DE FR GB IT LI NL SE**

(56) References cited :
US-A- 2 876 167
US-A- 3 137 632
US-A- 3 608 068
US-A- 3 678 154
US-A- 3 927 202
US-A- 4 244 931
ARCH. ORAL BIOL., vol. 15, 1970, Pergamon
Press, Great Britain F.J.DRAUS et al.
"Pyrophosphate and hexametaphosphate ef-
fects in vitro calculus formation"

(61) Publication number of the earlier application in
accordance with Art. 76 EPC : **0097476**

(73) Proprietor : **THE PROCTER & GAMBLE
COMPANY
One Procter & Gamble Plaza
Cincinnati Ohio 45202 (US)**

(72) Inventor : **Parran, John Joseph, Jr.
1155 Meredith Drive
Cincinnati Ohio 45231 (US)**
Inventor : **Sakkab, Nabil Yaqub
8297 Glenmill Road
Cincinnati Ohio 45249 (US)**

(74) Representative : **Brooks, Maxim Courtney et al
Procter & Gamble (NTC) Limited Whitley Road
Longbenton
Newcastle-upon-Tyne NE12 9TS (GB)**

EP 0 297 213 B1

## Description

The presente invention relates to use of soluble pyrophosphate salt in the manufacture of an oral composition for reducing the incidence of calculus on dental enamel, and to a process for reducing the incidence of calculus on dental enamel.

Dental calculus, or tartar as it is sometimes called, is a deposit which forms on the surfaces of the teeth at the gingival margin. Supragingival calculus appears principally in the areas near the orifices of the salivary ducts ; e.g., on the lingual surfaces of the lower anterior teeth and on the buccal surfaces of the upper first and second molars, and on the distal surfaces of the posterior molars.

Mature calculus consists of an inorganic portion which is largely calcium phosphate arranged in a hydroxyapatite crystal lattice structure similar to bone, enamel and dentine. An organic portion is also present and consists of desquamated epithelial cells, leukocytes, salivary sediment, food debris and various types of microorganisms.

As the mature calculus develops, it becomes visibly white or yellowish in color unless stained or discolored by some extraneous agent. In addition to being unsightly and undesirable from an aesthetic standpoint, the mature calculus deposits are constant sources of irritation of the gingiva.

A wide variety of chemical and biological agents have been suggested in the art to retard calculus formation or to remove calculus after it is formed. Mechanical removal of this material periodically by the dentist is, of course, routine dental office procedure.

The chemical approach to calculus inhibition generally involves chelation of calcium ion and/or crystal growth inhibition which prevents the calculus from forming and/or breaks down mature calculus by removing calcium.

The prior art discloses a number of chelating agents for this purpose. GB-A-490,384, February 15, 1937, discloses oral compositions containing ethylenediaminetetraacetic acid, nitrilotriacetic acid and related compounds as anticalculus agents. US-A-3,678,154, July 18, 1972 to Widder et al discloses oral compositions containing certain polyphosphonates and fluoride. US-A-3,737,533, June 5, 1973 to Francis discloses oral compositions containing certain carbonyl diphosphonates.

In addition to the above references, the prior art discloses dentifrices and mouthwashes containing soluble pyrophosphate salts which have been indicated for a variety of purposes. Included among such references are US-A-2,941,926, June 21, 1960 to Salzmann et al which discloses dental powders containing chlorophyll and pyrophosphate salts. US-A-3,137,632 dicloses toothphastes containing pyrophosphate salts. US-A-3,927,201 and US-A-3,927,202 disclose toothpastes which utilize soluble pyrophosphates as abrasives. US-A-4,244,931 and US-A-4,247,526 disclose pyrophosphate salts in dicalcium phosphate systems. Jap. Patent Application Disclosure No. 4945-1974 discloses soluble pyrophosphates in a variety of dentifrice systems. US-A-4,323,551 discloses tetraalkali metal salts in mouthwash compositions. Finally Draus, Lesniewski and Miklos, Pyrophosphate and Hexametaphosphate Effects In Vitro Calculus Formation, Arch. Oral Biol., Vol. 15, pp. 893-896, (1970) disclose the in vitro effectiveness of soluble pyrophosphate salts against calculus. However, they indicate that pyrophosphate would be inhibitied by pyrophosphatase in vivo.

In spite of the many disclosures in the anticalculus and pyrophosphate areas, the need for an effective anticalculus product still exists. Surprisingly mixtures of certain pyrophosphate salts can provide a safe and effective product while also not presenting difficult formulation problems.

It is an object of the present invention to provide an effective method for treating calculus.

This and other objects will become more clear from the detailed description which follows.

All percentages and ratios used herein are by weight unless otherwise specified.

Accordingly, the present invention relates to the use of a soluble pyrophosphate salt in the manufacture of an oral composition for reducing the incidence of calculus on dental enamel, the soluble pyrophosphate salt being used in an amount providing at least 1.5% by weight of the composition of pyrophosphate ions ($P_2O_7^=$), the composition additionally having a pH of from 6.0 to 10.0 and comprising a fluoride ion source in an amount sufficient to supply from 50 ppm to 3500 ppm of fluoride ions.

The present invention also encompasses a process for reducing the incidence of calculus on dental enamel.

The essential as well as optional components of the compositions manufactured and used according to the present invention are described in the following paragraphs :

## Dental Abrasive

The abrasives useful in the dentifrice compositions include many different materials. Calcium pyrophosphate, including the β-phase calcium pyrophosphate prepared in accordance with the teaching of US-A-3,112,247 can be used. The β-phase calcium pyrophosphate is prepared by heating γ-phase calcium pyrophosphate to 700-900°C. to change at least 50% of the γ-phase to β-phase and then immediately cooling. Another class of abrasives for use herein are the particulate thermosetting polymerized resins as described in US-A-3,070,510. Suitable resins include, for example, melamines, phenolics, ureas, melamine-ureas, melamineformaldehydes, urea-formaldehydes, melamine-urea-formaldehydes, cross-linked epoxides, and cross-linked polyesters.

Silica dental abrasives are also useful in the present compositions. The silica abrasive polishing material generally has an average particle size ranging between 0.1 and 30 μm preferably 5 and 15 μm. The abrasive can be precipitated silica or silica gels such as the silica xerogels described in US-A-3,538,230. Preferred are the silica xerogel marketed under the tradename "Syloid (RTm)"by the W. R. Grace & Company, Davison Chemical Division. Also preferred are the precipitated silica materials such as those marketed by the J. M. Huber Corporation under the tradename, "Zeodent (RTm)". The type of silica dental abrasives useful in the toothpastes of the present invention are described in more detail in US-A-3,862,307.

Other suitable abrasives include alumina, and the insoluble metaphosphates such as insoluble sodium metaphosphate (IMP). Mixtures of abrasives can also be used. In any case, the total amount of abrasive in the dentifrice embodiments of this invention can range from 0% to 70% by weight of dentifrice. Preferably, toothpastes contain from 10% to 50% by weight of abrasive.

The preferred abrasives are the β-phase calcium pyrophosphate of US-A-3,112,247 ; alumina ; insoluble metaphosphate ; the resinous abrasives of US-A-3,070,510 ; and the silica abrasives since they are more compatible with the agents. Most preferred are the silica abrasives.

## Fluoride Ion Source

The second essential component of the compositions is a fluoride ion source, The number of such sources is great and includes those disclosed in US-A-3,535,421. Typical materials include :

Stannous fluoride, potassium fluoride, lithium fluoride, cesium fluoride, ammonium fluoride, aluminum fluoride, cupric fluoride, indium fluoride, stannous fluorozirconate, lead fluoride, ferric fluoride, nickel fluoride, paladium fluoride, silver fluoride, zinc fluoride, zirconium fluoride, hexylamine hydrofluoride, laurylamine hydrofluoride, myristylamine hydrofluoride, decanolamine hydrofluoride, octadecenylamine hydrofluoride, myristoxyamine, hydrofluoride, diethylaminoethyloctoylamide hydrofluoride, diethanolamineoethyloleylamide hydrofluoride, diethanolaminopropyl-N'-octadecenylamine dihydrofluoride, 1-ethanol-2-hexadecylimidazoline dihydrofluoride, octoylethanolamine hydrofluoride, octyltrimethylammonium fluoride, dodecylethyldimethylammonium fluoride, tetraethylammonium fluoride, dilauryldimethylammonium fluoride. $\Delta^{8,9}$-octadecenylbenzyldimethylammonium fluoride, dioctyldiethylammonium fluoride, cyclohexylcetyldimethylammonium fluoride, furfuryllauryldimethyl-ammonium fluoride, phenoxyethylcetyldimethylammonium fluoride, N :N'-tetramethyl-N :N ;-dilaurylethylenediammonium difluoride, N-cetylpyridinium fluoride, N :N-dilauryl-morpholinium fluoride, N-myristyl-N-ethylmorpholinium fluoride, N-(octylaminocarbonylethyl)N-benzyl-dimethylammonium fluoride, N(β-hydroxydodecyl) trimethylammonium fluoride, N-phenyl-N-hexadecyldiethylammonium fluoride, N-cyclohexyl-N-octadecyldimethylammonium fluoride, N-(2-carbomethoxyethyl)-N-benzyldimethylammonium fluoride, N-(2-carbocyclohexoxyethyl)-N-myristyldimethylammonium fluoride, N-(2-carbobenzyloxyethyl)-N-dodecyldimethylammonium fluoride, N-[2-(N :N'-dimethylaminocarbonyl)-ethyl]-N-dodecyldiethylammonium fluoride, N-carboxymethyl-N-cicosyldimethylammonium fluoride, betaine hydrofluoride, sarcosine stannous fluoride, alanine stannous fluoride, glycine potassium fluoride, sarcosine potassium fluoride, glycine hydrofluoride, lysine hydrofluoride, alanine hydrofluoride, betaine zirconium fluoride, sodium monofluoro phosphate and mixtures thereof. Sodium fluoride is the preferred fluoride source.

The amount of the fluoride ion source should be sufficient to provide from 50 ppm to 3500 ppm, preferably from 500 ppm to 3000 ppm of fluoride ions.

## Di alkali Metal and Tetra alkali Metal Salts

The pyrophosphate salts useful herein include dialkali metal pyrophosphate and mixtures of the dialkali metal and tetraalkali metal pyrophosphate salts. $Na_2H_2P_2O^7$, $Na_4P_2O_7$ and $K_4P_2O_7$ in their unhydrated as

well as hydrated forms are the preferred species. The levels of each of these species which preferably are used in the compositions are as follows (all are in the unhydrated form).

$$Na_2H_2P_2O_7 \quad - \quad 0.5\% \quad - \quad 13.8\%$$
$$Na_4P_2O_7 \quad - \quad 0 \quad - \quad 6.0\%$$
$$K_4P_2O_7 \quad - \quad 0 \quad - \quad 4.0\%$$

The minimum amount of $P_2O_7^{-4}$ required in the present compositions, 1.5% can therefore be provided solely by $Na_2H_2P_2O_7$ or mixtures of $Na_2H_2P_2O_7$ with either or both of the tetra alkali metal salts. Preferred are binary mixtures of the sodium salts and ternary mixtures of those with the tetra potassium salt. The upper limits on the sodium species are determined by solubility considerations while the tetra potassium level is established for taste reasons.

The pyrophosphate salts are described in more detail in Kirk & Othmer, Encyclopedia of Chemical Technology, Second Edition, Volume 15, Interscience Publishers (1968).

Water

Water is another essential component of the compositions. Water employed in the preparation of commercially suitable oral compositions should preferably be of low ion content and free of organic impurities. Water comprises from 2% to 95%, preferably from 20% to 95% of the compositions of this invention. When in the form of toothpastes, the amount of water is preferably from 2% to 45%, while mouthwashes preferably contain from 45% to 95%.

Optional Components

In addition to the above described essential components, the oral compositions can contain a variety of optional conventional oral composition components. Such optional ingredients include sudsing agents, flavoring agents, sweetening agents, antiplaque agents, coloring agents, and pigments.

A preferred optional ingredient is a sudsing agent. Suitable sudsing agents are those which are reasonably stable and form suds throughout a wide pH range, i.e., non-soap anionic, nonionic, cationic, zwitterionic and amphoteric organic synthetic detergents. Sudsing agents of these types are described more fully in US-A-3,959,458 and US-A-3,937,807.

Anionic sudsing agents useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate and sodium coconut monoglyceride sulfonates are examples of anionic surfactants of this type. Mixtures of anionic surfactants can also be employed.

The nonionic sudsing agents useful herein can be broadly defined as compounds produced by the condensation of alkylene oxide groups (hyrophilic in nature) with an organic hydrophobic compound which may be aliphatic or alkylaromatic in nature. Examples of suitable nonionic sudsing agents include the Pluronics (RTm), polyethylene oxide condensate of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

The zwitterionic synthetic sudsing agents useful herein can be broadly described as derivatives of aliphatic quaternary ammonium, phosphonium, and sulfonium compounds in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate, orphosphonate.

The cationic susding agents useful herein can be broadly defined as quaternary ammonium compounds having one long alkyl chain containing from about 8 to about 18 carbon atoms such as lauryl trimethylammonium chloride; cetyl pyridinium chloride, cetyl trimethylammonium bromide; di-isobutylphenoxyethoxyethyl-dimethylbenzylammonium chloride; coconutalkyltrimethylammonium nitrite; cetyl pyridinium fluoride; etc.

The amphoteric susding agents useful herein can be broadly described as derivatives of aliphatic secondary and tertiary amines in which the aliphatic radical can be straight chain or branched and wherein one of the aliphatic substituents contains from about 8 to about 18 carbon atoms and one contains an anionic water-solubilizing group, e.g., carboxylate, sulfonate, sulfate, phosphate, or phosphonate.

The sudsing agent can be present in an amount from 0% to 10% by weight of the total composition.

Flavoring agents can also be added. Suitable flavoring agents include oil of wintergreen, oil of peppermint, oil of spearmint, oil of sassafras, and oil of clove. Sweetening agents which can be used include saccharin, dextrose, levulose, aspartame, D-tryptophan, dihydrochalcones, acesulfame and sodium cyclamate. Flavoring agents are generally used in the compositions at levels of from 0.4% to 2% by weight and sweetening agents at levels of from 0.1% to 5% by weight.

Binders can also be used in the toothpastes herein. Such binders include, for example, xanthan gum, carrageenan (Irish moss, Viscarin (RTm)), and carboxyvinyl polymers. These binders arte generally present at a level of from 0.1% to 1%.

Bis-biguanide antiplaque agents can also optionally be added. Such agents include chlorhexidine 1,6-bis [$N^5$-p-chlorophenyl-$N^1$-biguanido]hexane), the soluble and insoluble salts thereof and related materials such as 1,2-bis($N^5$-p-trifluoromethylphenyl-$N^1$-biguanido)ethane are described more fully in US-A-3,923,002, US-A-3,937,807, BE-A-843,244, and BE-A-844,764.

If present, the optional antiplaque agents generally comprise from 0% to 5% by weight of composition.

Another optional component is a humectant. The humectant serves to keep the toothpaste compositions from hardening upon exposure to air and in mouthwashes give a moist feel to the mouth. Certain humectants can also impart desirable sweetness of flavor to mouthwash and toothpaste compositions. The humectant, on a pure humectant basis, generally comprises from 0% to 70%, preferably from 0% to 55%, by weight of composition.

Suitable humectants include edible polyhydric alcohols such as glycerine, sorbitol, xylitol and propylene glycol. Sorbitol is frequently employed as a 70% aqueous solution known as Sorbo (RTm).

The mouthwashes may also contain ethanol in an amount of from abut 0 to about 30%.

The pH of the compositions is in the range of 6.0 to 10.0, preferably from 7.3 to 9.0. The pH is preferably achieved through a proper balancing of the pyrophosphate salts or by the addition of an alkaline or acidic agent.

## Method of Manufacture

The compositions herein are made using conventional mixing techniques. A typical method is described in Example I.

## Industrial Applicability

The compositions of the present invention are used in a conventional manner.

The following examples further describe and demonstrate preferred embodiments within the scope of the present invention.

## EXAMPLE I

The following is a toothpaste comprising a pyrophosphate salt for use according to the present invention.

| Component | % |
|---|---|
| Distilled Water | .16.484 |
| Sorbitol (70% Aqueous Solution) | 49.563 |
| Sodium Saccharin | 0.300 |
| Dye Solution | 0.350 |
| Precipitated Silica | 20.00 |
| Sodium Fluoride | 0.243 |
| Flavor | 1.330 |
| Sodium Alkyl Sulfate (27.9% Aqueous Solution) | 5.000 |
| Carbopol (RTm) 940s* | 0.180 |
| Xanthan Gum | 0.600 |
| $Na_4P_2O_7$ | 2.400 |
| $Na_2H_2P_2O_7$ | 1.190 |
| $K_4P_2O_7$ (63.5% Aqueous Solution) | 2.360 |
| | 100.000% |

* A carboxy vinyl polymer offered by B. F. Goodrich Company.

The above composition was made by combining the water and part of the sorbitol in an agitated mixture and heating this mixture to 60°C (140°F). The $Na_2H_2P_2O_7$, $Na_4P_2O_7$ saccharin, sodium fluoride and precipitated silica were then added in order and the total mixture was mixed for from 5 to 10 minutes. The flavor, dye and surfactant were then added. In a separate vessel the remainder of the sorbitol, the Carbopol (RTm) and the xanthan gum were slurried together and then added to the main mix tank. The complete batch was mixed for about one-half hour and subsequently milled and deaerated.

## EXAMPLE II

The following is another representative toothpaste comprising a pyrophosphate salt for use according to the present invention.

6

| Component | % |
|---|---|
| Sorbitol (70% Aqueous Solution) | 50.743 |
| Distilled Water | 16.484 |
| Sodium Saccharin | 0.300 |
| Dye Solution | 0.350 |
| Precipitated Silica | 20.000 |
| Sodium Fluoride | 0.243 |
| Flavor | 1.330 |
| Sodium Alkyl Sulfate(27.9% Aqueous Solution) | 5.000 |
| Carbopol (RTm) 940S | 0.180 |
| Xanthan Gum | 0.600 |
| $Na_4P_2O_7$ | 3.400 |
| $Na_2H_2P_2O_7$ | 1.370 |
| | 100.000% |

Both the composition of Example I and that of Example II are effective in reducing calculus and possess acceptable cosmetic properties.

In addition to the levels and combinations of ingredients shown in these examples, others can be used which are consistent with the invention disclosed and claimed herein.

## Claims

1. Use of a soluble pyrophosphate salt in the manufacture of an oral composition for reducing the incidence of calculus on dental enamel, the soluble pyrophosphate salt being used in an amount providing at least 1.5% by weight of the composition of pyrophosphate ions ($P_2O_7^-$), the composition additionally having a pH of from 6.0 to 10.0 and comprising a fluoride ion source in an amount sufficient to supply from 50 ppm to 3500 ppm of fluoride ions.

2. Use of a soluble pyrophosphate salt according to Claim 1 wherein the pyrophosphate salt is selected from dialkali metal pyrophosphate salts and mixtures of dialkali metal and tetraalkali metal pyrophosphate salts.

3. Use of a soluble pyrophosphate salt according to Claim 1 or 2 wherein the composition is in the form of a dentifrice which in addition contains a silica dental abrasive.

4. Use of a soluble pyrophosphate salt according to Claim 1 or 2 wherein the composition is in the form of a mouthwash.

5. Use of a soluble pyrophosphate salt according to Claim 1 or 2 wherein the composition is in the form of a liquid dentifrice.

6. A process for reducing the incidence of calculus on dental enamel comprising contacting the enamel surfaces in the mouth with a composition having a pH of from 6.0 to 10.0, the composition comprising a soluble pyrophosphate salt capable of providing at least 1.5% by weight of the composition of pyrophosphate ions ($P_2O_7^-$) and a fluoride ion source in an amount sufficient to supply from 50 ppm to 3500 ppm of fluoride ions.

## Ansprüche

1. Verwendung eines löslichen phosphatsalzes in der Herstellung einer oralen Zusammensetzung zur Veminderung des Auftretens von Zahnstein auf Zahnschmelz, wobei das lösliche Pyrophosphatsalz in einer Menge verwendet wird, die wenigstens 1,5 Gew.-% der Zusammensetzung an Pyrophosphationen $(P_2O_7^-)$ liefert, wobei die Zusammensetzung überdies einen pH-Wert von 6,0 bis 10,0 aufweist und eine Fluoridionenquelle in einer Menge umfaßt, die ausreicht, um 50 ppm bis 3.500 ppm Fluoridionen zu liefern.

2. Verwendung eines löslichen Pyrophosphatsalzes nach Anspruch 1, worin die Pyrophosphatsalz unter Dialkalimetallpyrophosphatsalzen und Gemischen aus Dialkalimetall- und Tetraalkalimetallpyrophosphatsalzen ausgewählt ist.

3. Verwendung eines löslichen Pyrophosphatsalzes nach Anspruch 1 oder 2, worin die Zusammensetzung in Form eines Zahnpflegemittels vorlieft, das zusätzlich ein Kieselsäure-Zahnchleifmittel enthält.

4. Verwendung eines löslichen Pyrophosphatsalzes nach Anspruch 1 oder 2, worin die Zusammensetzung in Form eines Mundwassers vorliegt.

5. Verwendung eines löslichen Pyrophophatsalzes nach Anspruch 1 oder 2, worin die Zusammensetzung in Form eines flüssigen Zahnpflegemittels vorliegt.

6. Verrfahren zur Verminderung des Auftretens von Zahnstein auf Zahnschmelz, umfassend das In-Berührung-Bringen der Zahnschmelzoberflächen im Mund mit einer einen pH-Wert von 6,0 bis 10,0 aufweisenden Zusammensetzung, welche Zusammensetzung ein lösliches Pyrophosphatsalz, das zur Lieferung von wenigstens 1,5 Gew.-% der Zusammensetzung an Pyrophosphationen $(P_2O_7^-)$ befähigt ist, und eine Fluoridionenquelle in einer zur Lieferung von 50 ppm bis 3.500 ppm an Fluoridionen ausreichenden Menge umfaßt.

## Revendications

1. Utilisation d'un sel de pyrophosphate soluble dans la fabrication d'une composition orale pour réduire l'apparition du tartre sur l'émail dentaire, le sel de pyrophosphate soluble étant utilisé en une quantité apportant au moins 1,5%, du poids de la composition, d'ions pyrophosphate $(P_2O_7^-)$, la composition ayant en outre un pH de 6,0 à 10,0 et comprenant une source d'ions fluorure, en une quantité suffisante pour apporter de 50 ppm à 3500 ppm d'ions fluorure.

2. Utilisation d'un sel de pyrophosphate soluble selon la revendication 1, dans laquelle le sel de pyrophosphate est choisi parmi les sels de pyrophosphates dialcalins et les mélanges de sels de pyrophosphates dialcalins et tétraalcalins.

3. Utilisation d'un sel de pyrophosphate soluble selon la revendication 1 ou 2, dans laquelle la composition se présente sous forme d'un dentifrice qui contient, en outre, un abrasif dentaire à base de silice.

4. Utilisation d'un sel de pyrophosphate soluble selon la revendication 1 ou 2, dans laquelle la composition se présente sous forme d'un bain de bouche.

5. Utilisation d'un sel de pyrophosphate soluble selon la revendication 1 ou 2, dans laquelle la composition se présente sous forme d'un dentifrice liquide.

6. Procédé de réduction de l'apparition du tartre sur l'émail dentaire, comprenant la mise en contact des surfaces de l'émail dans la bouche avec une composition ayant un pH de 6,0 à 10,0, la composition comprenant un sel de pyrophosphate capable d'apporter au moins 1,5%, du poids de la composition, d'ions pyrophosphate $(P_2O_7^-)$, et une source d'ions fluorure, en une quantité suffisante pour apporter de 50 ppm à 3500 ppm d'ions fluorure.